# EUROPÄISCHE PATENTSCHRIFT

(11) **EP 3 319 931 B1**
(45) Veröffentlichungstag und Bekanntmachung des Hinweises auf die Patenterteilung: **30.10.2019**
(21) Anmeldenummer: 16735664.1
(22) Anmeldetag: 06.07.2016
(51) Int. Cl.: C07C 51/41, C07C 53/122

(54) **VERFAHREN ZUR HERSTELLUNG VON CALCIUMDIPROPIONAT**
METHOD FOR PRODUCING CALCIUM DIPROPIONATE
PROCÉDÉ DE PRÉPARATION DE DIPROPIONATE DE CALCIUM

(30) Priorität: 10.07.2015 DE 102015212984
(43) Veröffentlichungstag der Anmeldung: 16.05.2018
(73) Patentinhaber: Addcon Europe GmbH, 06749 Bitterfeld-Wolfen (DE)
(72) Erfinder: EDERLE-LERCH, Klaus, 83229 Aschau im Chiemgau (DE); KILLIAN, Norman, 06114 Halle (Saale) (DE); KOCHANNEK, Bernd, 53113 Bonn (DE); STÜWE, Hans-Jürgen, 06905 Bad Schmiedeberg (DE)
(74) Vertreter: dompatent von Kreisler Selting Werner - Partnerschaft von Patent- und Rechtsanwälten mbB
(86) Internationale Anmeldenummer: PCT/EP2016/066019
(87) Internationale Veröffentlichungsnummer: WO 2017/009151

(56) Entgegenhaltungen:
- EP-A2- 1 072 581
- WO-A1-03/087028
- US-A1- 2003 032 841

## Beschreibung

Gegenstand der Erfindung ist ein Feststoff-Verfahren zur Herstellung von Calciumdipropionat.

Calciumdipropionat, gelegentlich auch als Calciumpropionat bezeichnet, (Summenformel: C₆H₁₀CaO₄; CAS No. 4075-81-4) wird sowohl als Lebensmittelzusatzstoff (E 282) als auch bei der Formulierung von Futtermitteladditiven weltweit in industriellem Maßstab eingesetzt. Dazu wird typischerweise Calciumhydroxid in wässriger Suspension - entweder direkt aus Calciumoxid durch Hydratisierung hergestellt oder durch Suspendieren von Calciumhydroxid in Wasser - mit Propionsäure neutralisiert. Nach vollständiger Neutralisation löst sich das entstandene Calciumdipropionat im heißen Wasser auf und kann zur Entfernung von Verunreinigungen einer Klärfiltration unterzogen und darauffolgend aufkonzentriert werden. Die Abtrennung von Calciumdipropionat aus der wässrigen Lösung erfolgt entweder durch Eindampfung mit anschließender Abtrennung der gebildeten Kristalle in herkömmlichen Fest-/Flüssig Trennsystemen oder durch Sprühtrocknung. Diese Technologie führt zu Produkten mit hoher Reinheit, ist aber, nicht zuletzt aufgrund der niedrigen Wasserlöslichkeit von Calciumdipropionat, verbunden mit einem hohen Energiebedarf bei der Aufarbeitung und einer hohen Komplexität der benötigten Produktionsanlagen, sehr kostenintensiv.

Eine Vielzahl der bekannten Verfahren zur Herstellung von Alkali- und Erdalkalipropionaten basieren auf der Neutralisation von Alkali- und/ oder Erdalkalihydroxid in wässriger Phase mit Propionsäure. Die Verfahren werden drucklos oder unter erhöhtem Druck mit Propionsäure wasserfrei oder mit wässriger Propionsäure durchgeführt. DE 32 15 752 A1/ EP 0 093 317 A1/ US-Patent 4,700,000 beschreibt beispielhaft ein solches Verfahren. Die entstehenden Reaktionslösungen werden typischerweise durch Klärfiltration gleichfalls aufgrund der mäßigen Wasserlöslichkeit von Calciumdipropionat unter erhöhtem Druck und Temperatur gereinigt. Die Abtrennung und Aufarbeitung der erzeugten Alkali- und/ oder Erdalkalipropionate erfolgt in unterschiedlichen Trennverfahren. Im Falle von Calciumdipropionat wird eine wesentliche Menge des aktuell erzeugten Produktes durch ein Sprühtrocknungsverfahren aus der gereinigten Calciumdipropionat-Lösung oder aus einer Calciumdipropionat-Suspension erzeugt. Darüber hinaus hat auch die Eindampfung der Reaktionslösung und die Kristallisation von Calciumdipropionat aus der gesättigten Lösung mit anschließender Abtrennung durch Zentrifugierung eine große Bedeutung bei den derzeit angewendeten Technologien. Weiterhin wird auch die direkte Umsetzung von Calciumoxid und/ oder Calciumhydroxid mit Propionsäure in Reaktoren ohne Wasserzusatz praktiziert. Die Umsetzung findet dabei ausschließlich in der festen Phase statt. Probleme bei der Abführung der **Reaktionswärme** führen zu lokalen Überhitzungen, Zersetzungen und damit zur Farbveränderungen (Vergrauung) des Calciumdipropionats sowie zu Verlusten von Propionsäure durch die sich **bildenden** Azeotrope bei noch nicht vollständiger Umsetzung. Diese Gründe und die inhomogene Reaktionsführung, bedingt durch die Exothermie der Neutralisation. Die daraus resultierenden Qualitätsunterschiede führen bei den marktüblichen Calciumdipropionat-Produkten dazu, dass diese Feststoff-Verfahren nicht in großem Umfang praktiziert werden.

EP 1 072 581 A2 beschreibt ein Verfahren zur Herstellung von Calciumpropionat und eine Vorrichtung zur Durchführung des Verfahrens. Beschrieben wird ein Verfahren zur Herstellung von pulverförmigem Calciumpropionat unter Verwendung eines konvektiven Trockners, bei dem eine einteilige, pumpbare Suspension von Calciumpropionat in Wasser mit einem Calciumpropionat-Gehalt von größer oder gleich 30 % (w/w) einem konvektiven Trockner zugeführt wird sowie eine Vorrichtung zur Durchführung des Verfahrens mit einem Reaktor, einer Schlaufe, einer Dispergiermaschine und gegebenenfalls einem Vorlagebehälter mit einem Trockner.

In US 6,986,909 B2 wird der Versuch beschrieben, das Problem der schlechten Wärmeabfuhr durch das Auftragen der Propionsäure auf einen anorganischen inerten Carrier zu lösen, der bei der folgenden Umsetzung durch Zugabe von Calciumhydroxid eine günstigere Wärmeverteilung ermöglichen soll. Der inerte Carrier verbleibt aber dann im Endprodukt, welches demzufolge nur für Futtermittelapplikationen geeignet ist.

Die Aufgabe der vorliegenden Erfindung besteht in der Bereitstellung eines Feststoff-Verfahrens zur Herstellung von Calciumdipropionat ohne dass die freiwerdende Reaktionsenergie und das im Fall der Verwendung von Calciumcarbonat entstehende Kohlendioxid zu Verlusten an Propionsäure, zu sehr langen Reaktionszeiten aufgrund der schwierigen Kühlbarkeit von Feststoffen, zu der bei Feststoffverfahren oft zu beobachtenden mangelnden Homogenität des Endproduktes und zu Energieverlusten führt

Die vorgenannte Aufgabe wird in einer ersten Ausführungsform gelöst durch ein carrierfreies Feststoff-Verfahren zur Herstellung von pulverförmigem Calciumpropionat in einem druckfesten Feststoffmischreaktor durch Vorlage von Calciumoxid, Calciumhydroxid und Calciumcarbonat oder Gemischen dieser Calciumverbindungen und Zugabe von Propionsäure durch Umsetzung direkt in der Feststoffphase bei einem gegenüber Normaldruck erhöhten Druck.

Die vorliegende Erfindung beschreibt ein carrierfreies Feststoff-Verfahren, bei dem Propionsäure unter Druck in einem entsprechenden Reaktor mit Gemischen aus Calciumoxid, Calciumoxid und Calciumhydroxid, Calciumoxid und Calciumcarbonat oder den jeweiligen reinen Calciumverbindungen direkt in der Feststoffphase zu Calciumdipropionat umgesetzt werden kann, ohne dass die freiwerdende Reaktionsenergie und das im Fall der Verwendung von Calciumcarbonat entstehende Kohlendioxid zu Verlusten an Propionsäure, zu sehr langen Reaktionszeiten aufgrund der schwierigen Kühlbarkeit von Feststoffen, zu der bei Feststoffverfahren oft zu beobachtenden mangelnden Homogenität des Endproduktes und zu Energieverlusten führt, da die freiwerdende Reaktionsenergie durch die Druckfahrweise im System verbleibt und nach abgeschlossener Reaktion unmittelbar zur Trocknung des Endproduktes genutzt werden kann.

In den bisher praktizierten Feststoff-Verfahren zur Herstellung von Calciumdipropionat durch direkte Umsetzung von Calciumoxid oder Gemischen aus Calciumoxid und Calciumhydroxid und Propionsäure ist in der Anfangsphase eine sehr effiziente Kühlung notwendig, um einen Temperaturanstieg, der zu Verlusten an Propionsäure und zu Produktzersetzung führt, zu verhindern. Nachdem das im Reaktor vorhandene Calciumoxid durch gebildetes Reaktionswasser hydrolisiert ist, klingt die Wärmeentwicklung spürbar ab. Die durch die Neutralisation von Calciumhydroxid gebildete Wärme reicht in der Praxis nicht mehr aus, um das sich bildende Reaktionswasser vollständig zu verdampfen. Es muss also durch Wärmezufuhr die Reaktionsführung sowie die Trocknung vollständig abgeschlossen werden. In dem erfindungsgemäßen Verfahren wird daher in einem druckfesten Edelstahl-Reaktor eine Mischung aus Calciumoxid und Calciumhydroxid oder eine Mischung aus Calciumoxid und Calciumcarbonat vorgelegt. Unter intensivem Mischen wird dann die stöchiometrisch notwendige Propionsäure in den Reaktor eindosiert, Temperatur und Druck steigen dabei deutlich an. Die Temperatur kann 150 °C bis 180 °C erreichen, der Druck steigt je nach den Größenverhältnissen zwischen Reaktorvolumen und der im Reaktor vorhandenen Einsatzstoffmenge auf 2 bis 10 bar. Nach vollständiger Propionsäure-Zugabe beginnt eine etwa ½ bis 2 stündige Nachreaktionszeit. Im Anschluss daran wird der Reaktorinhalt kontrolliert entspannt. Es entweicht in Abhängigkeit von den Einsatzstoffen und/ oder den Einsatzstoffmengen ein Wasserdampf- und/ oder ein Wasserdampf-/Kohlendioxidgemisch, das noch Spuren von Calciumhydroxid-Staub oder von Propionsäure enthalten kann. Je nach berechnetem Stoffmengen-Verhältnis von Calciumoxid zu Calciumhydroxid beziehungsweise Calciumoxid zu Calciumcarbonat kann die Reaktion so gesteuert werden, dass keine zusätzliche Energie zur vollständigen Trocknung des gebildeten Calciumdipropionats mehr zugeführt werden muss. Es wird ein homogenes Produkt erhalten, welches den üblichen Reinheitskriterien für Calciumdipropionat für Lebensmittel- oder Futtermittelzwecke entspricht und homogen bezüglich pH-Wert und Partikelgrößenverteilung hergestellt werden kann.

### Ausführungsbeispiele:

### Beispiel 1 - Herstellung von Calciumdipropionat aus Calciumoxid sowie Propionsäure

In einem 110 L Edelstahl-Reaktor wurden 15,8 kg Calciumoxid vorgelegt. Über einen Zeitraum von 4 Stunden wurden 41,7 kg Propionsäure zudosiert. Gegen Ende der Zugabe der Propionsäure musste durch vorsichtiges Entspannen die Reaktorinnentemperatur auf 180 °C und der Reaktordruck auf 5 bar gehalten werden. Das Reaktionsgemisch verblieb danach zur vollständigen Umsetzung noch 30 Minuten im Reaktor. Anschließend wurde durch vorsichtiges und kontrolliertes Entspannen das bei der Reaktion entstandene Wasser aus dem Reaktor über einen Brüdenfilter verdampft. Nachdem dabei die Reaktortemperatur unter den Taupunkt sank, konnte durch Nachheizen und Anlegen von Vakuum das Reaktionsprodukt bis zum gewünschten Wassergehalt getrocknet werden. Als Reaktionsprodukt entstanden 52,1 kg Calciumdipropionat als feines weißes Pulver mit einem Feuchtigkeitsgehalt von 1,3 Gew.-% und einem Anteil von 0,48 Gew.-% an wasserunlöslichen Verbindungen. Der Reingehalt des getrockneten Produktes betrug 96,2 Gew.-%.

### Beispiel 2 - Herstellung von Calciumdipropionat aus einem Gemisch aus Calciumoxid und Calciumhydroxid sowie Propionsäure

In einem 110 L Edelstahl-Reaktor wurden 9,2 kg Calciumoxid und 8,6 kg Calciumhydroxid vorgelegt. Über einen Zeitraum von 2 Stunden wurden 41,5 kg Propionsäure zudosiert. Am Ende der Zugabe der Propionsäure waren die Reaktorinnentemperatur auf 180 °C und der Reaktordruck auf 5 bar angestiegen. Das Reaktionsgemisch verblieb danach zur vollständigen Umsetzung noch 30 Minuten im Reaktor. Anschließend wurde durch vorsichtiges Entspannen das bei der Reaktion entstandene Wasser aus dem Reaktor über einen Brüdenfilter verdampft. Nachdem dabei die Reaktortemperatur unter den Taupunkt sank, konnte durch Nachheizen und Anlegen von Vakuum das Reaktionsprodukt bis zum gewünschten Wassergehalt getrocknet werden. Als Reaktionsprodukt entstanden 54,0 kg Calciumdipropionat als feines weißes Pulver. Das erzeugte Calciumdipropionat hatte einen Feuchtigkeitsgehalt von 1,3 Gew.-% und einem Anteil von 0,55 Gew.-% an wasserunlöslichen Verbindungen. Der Reingehalt des getrockneten Produktes betrug 96,1 Gew.-%.

### Beispiel 3 - Herstellung von Calciumdipropionat aus einem Gemisch aus Calciumoxid und Calciumcarbonat sowie Propionsäure

In einem 110 L Edelstahl-Reaktor wurden 9,2 kg Calciumoxid und 11,7 kg Calciumcarbonat vorgelegt. Über einen Zeitraum von 2 Stunden wurden 41,5 kg Propionsäure zudosiert. Am Ende der Zugabe der Propionsäure waren die Reaktorinnentemperatur auf 160 °C und der Reaktordruck auf 3 bar angestiegen. Das Reaktionsgemisch verblieb danach zur vollständigen Umsetzung noch 300 Minuten im Reaktor. Anschließend wurde durch vorsichtiges Entspannen das bei der Reaktion entstandene Kohlendioxid und das gebildete Wasser aus dem Reaktor über einen Brüdenfilter verdampft. Nachdem dabei die Reaktortemperatur unter den Taupunkt sank, konnte durch Nachheizen und Anlegen von Vakuum das Reaktionsprodukt bis zum gewünschten Wassergehalt getrocknet werden. Als Reaktionsprodukt entstanden 54 kg Calciumdipropionat als feines weißes Pulver mit einem Reingehalt von 96,6 Gew.-% und einem Anteil an wasserunlöslichen Verbindungen in Höhe von 0,4 Gew.-% und einem Feuchtigkeitsgehalt von 1,6 Gew.-%.

### Beispiel 4 - Herstellung von Calciumdipropionat aus Calciumcarbonat sowie Propionsäure

In einem 110 L Edelstahl-Reaktor werden 28,2 kg gemahlenes Calciumcarbonat vorgelegt. Über einen Zeitraum von 2 Stunden wurden 41,5 kg Propionsäure zudosiert. Am Ende der Zugabe der Propionsäure waren die Reaktorinnentemperatur auf 155 °C und der Reaktordruck auf 3 bar angestiegen. Das Reaktionsgemisch verblieb danach zur vollständigen Umsetzung noch 360 Minuten im Reaktor. Anschließend wurde durch vorsichtiges Entspannen das bei der Reaktion entstandene Kohlendioxid und das gebildete Wasser aus dem Reaktor über einen Brüdenfilter verdampft. Nachdem dabei die Reaktortemperatur unter den Taupunkt sank, konnte durch Nachheizen und Anlegen von Vakuum das Reaktionsprodukt bis zum gewünschten Wassergehalt getrocknet werden. Als Reaktionsprodukt entstanden 54 kg Calciumdipropionat als feines weißes Pulver mit einem Reingehalt von 96,2 Gew.-%, einem Anteil von 0,4 Gew.-% an wasserunlöslichen Verbindungen und einem Feuchtigkeitsgehalt von 1,5 Gew.-%.

## Patentansprüche

1. Carrierfreies Feststoff-Verfahren zur Herstellung von pulverförmigem Calciumpropionat in einem druckfesten Feststoffmischreaktor durch Vorlage von Calciumoxid, Calciumhydroxid und Calciumcarbonat oder Gemischen dieser Calciumverbindungen und Zugabe von Propionsäure durch Umsetzung direkt in der Feststoffphase bei einem gegenüber Normaldruck erhöhten Druck.

2. Verfahren nach Anspruch 1, **dadurch gekennzeichnet, dass** man die Temperatur während **der** Umsetzung auf einen Bereich von 120 bis 180 °C einstellt.

3. Verfahren nach Anspruch 1 oder 2, **dadurch gekennzeichnet, dass** man den Überdruck im Reaktor auf bis zu 10 bar ansteigen läßt.

4. Verfahren nach einem der Ansprüche 1 bis 3, **dadurch gekennzeichnet, dass** man die Umsetzung im Bereich von 0,5 bis 3 h durchführt.

5. Verfahren nach einem der Ansprüche 1 bis 4, **dadurch gekennzeichnet, dass** man im Anschluss an die Umsetzung durch Nachheizen und Vakuum das entstandene Produkt trocknet.

6. Verfahren nach Anspruch 5, **dadurch gekennzeichnet, dass** man die Stoffmengenverhältnisse von Calciumoxid zu Calciumhydroxid oder Calciumoxid zu Calciumcarbonat so einstellt, dass die entstehende Reaktionswärme ausreicht, um die Trocknung des Produktes ohne zusätzliche Zufuhr von Heizenergie zu gewährleisten.

## Claims

1. A carrier-free solid-material method for the production of powdery calcium propionate in a pressure-resistant solid-state mixing reactor by charging calcium oxide, calcium hydroxide or calcium carbonate or mixtures of these calcium compounds, and adding propionic acid by a reaction directly in the solid phase under a pressure that is increased above normal pressure.

2. The process according to claim 1, **characterized in that** the temperature during the reaction is adjusted within a range of from 120 to 180 °C.

3. The process according to claim 1 or 2, **characterized in that** the overpressure in the reactor is allowed to increase to up to 10 bar.

4. The process according to any of claims 1 to 3, **characterized in that** the reaction is performed for a time within a range of from 0.5 to 3 hours.

5. The process according to any of claims 1 to 4, **characterized in that** the formed product is dried following the reaction by further heating and applying a vacuum.

6. The process according to claim 5, **characterized in that** the molar ratios of calcium oxide to calcium hydroxide or calcium oxide to calcium carbonate are set to such values that the released reaction heat is sufficient to ensure drying of the product without additional input of heating energy.

## Revendications

1. Procédé à l'état solide et exempt de support pour préparer du propionate de calcium pulvérulent dans un réacteur de mélange à l'état solide, résistant à la pression, consistant à charger de l'oxyde de calcium, de l'hydroxyde de calcium et du carbonate de calcium, ou des mélanges de ces composés de calcium, et ajouter de l'acide propionique en faisant réagir directement dans la phase solide sous une pression élevée par rapport à la pression normale.

2. Procédé selon la revendication 1, **caractérisé en ce que** la température est réglée dans une gamme comprise entre 120 et 180 °C pendant la réaction.

3. Procédé selon la revendication 1 ou 2, **caractérisé en ce que** la pression positive dans le réacteur est laissée s'élever jusqu'à 10 bar.

4. Procédé selon l'une quelconque des revendications 1 à 3, **caractérisé en ce que** la réaction est effectuée pendant une durée comprise entre 0,5 et 3 heures.

5. Procédé selon l'une quelconque des revendications 1 à 4, **caractérisé en ce que** le produit formé est séché après la réaction en continuant le chauffage et appliquant un vide.

6. Procédé selon la revendication 5, **caractérisé en ce que** les rapports molaires de l'oxyde de calcium à l'hydroxye de calcium ou de l'oxyde de calcium au carbonate de calcium sont réglés de manière que la chaleur de réaction produite soit suffisante pour garantir le séchage du produit sans apport additionnel d'énergie de chauffage.
